# EUROPÄISCHE PATENTSCHRIFT

(11) **EP 1 449 509 B1**
(45) Veröffentlichungstag und Bekanntmachung des Hinweises auf die Patenterteilung: **23.08.2006**
(21) Anmeldenummer: 04002523.1
(22) Anmeldetag: 05.02.2004
(51) Int. Cl.: A61K 8/00, A61K 8/11, A61Q 19/00

(54) **Kosmetische Zubereitungen mit Wirkstoffen in Mikrokapseln**
cosmetic compositions containing active agents in microcapsules
compositions cosmétiques contenant des agents actifs dans des microcapsules

(30) Priorität: 18.02.2003 DE 10306604
(43) Veröffentlichungstag der Anmeldung: 25.08.2004
(73) Patentinhaber: Goldschmidt GmbH, 45127 Essen (DE)
(72) Erfinder: Jungmann, Nadja, Dr., 45144 Essen (DE); Lersch, Peter, Dr., 46537 Dinslaken (DE); Schick, Ute, Dr., 50676 Köln (DE); Schiemann, Yvonne, 45359 Essen (DE); Weitemeyer, Christian, Dr., 45134 Essen (DE)

(56) Entgegenhaltungen:
- EP-A- 1 222 918
- WO-A-97/47288
- DE-A- 10 008 305
- FR-A- 2 753 639
- US-A- 5 364 634
- DATABASE CAPLUS [Online] CHEMICAL ABSTRACTS SERVICE, COLUMBUS, OHIO, US; XP002252137 gefunden im STN Database accession no. 1995:634746 & JP 07 096166 A (SUZUKI YUSHI KOGYO KK) 11. April 1995 (1995-04-11)
- PATENT ABSTRACTS OF JAPAN Bd. 012, Nr. 306 (C-522), & JP 63 079818 A (TSUMURA JUNTENDO INC), 9. April 1988 (1988-04-09)

## Beschreibung

Gegenstand der Erfindung sind kosmetische Zubereitungen enthaltend einen oder mehrere Wirkstoffe in einer Mikroverkapselung deren Verkapselungsmaterial im pH-Bereich der Haut durchlässig und/oder abgebaut wird, welche dadurch gekennzeichnet sind, dass das Kernmaterial frei von porösen Materialien ist und deren Verkapselungsmaterial sich bei ph-Werten von zwischen ca. 4,6 bis ca. 6,0 zersetzt.

Wenn es darum geht, besondere Wirkungen von Kosmetikprodukten zu erreichen und auszuloben, sind die Inhaltsstoffe ein zentrales Thema. Das hohe Niveau der angebotenen Inhalts- und Rohstoffe in kosmetischen Formulierungen wird kontinuierlich erweitert, da Verbraucher an anspruchsvollen und wirksamen Produkten interessiert sind, welche den Effekten des Älterwerdens entgegenwirken können. Dabei richtet sich das Interesse der Kosmetikhersteller auch auf Wirkstoffe, die in der Lage sind die Haut zu revitalisieren oder vor den Folgen der Lichtalterung zu schützen. Dienten solche Stoffe in der Vergangenheit primär der Glättung und Feuchtigkeitsbildung der Haut, so werden sie heute durch eine Vielzahl unterschiedlicher Materialien mit physiologischer Wirkung ergänzt. Beispiele hierfür sind Vitamine, Fruchtsäuren oder auch Ceramide. Hierbei ist auch die Art und Weise wie solche Wirkstoffe stabilisiert werden von zunehmender Bedeutung. In der Kosmetik besteht ein hohes Interesse an Wirkstoffen, die in wässriger oder auch in wasserhaltigen Systemen stabil gelagert werden können.

Es ist zum Zwecke der Anwendung eines oder mehrerer kosmetischer Hautwirkstoffe und/oder Aromastoffe und/oder Nahrungsergänzungsmittel wünschenswert, diese zu verkapseln oder mit einem Überzug zu versehen. Insbesondere ist diese Maßnahme für thermolabile, oxidationsempfindliche Stoffe oder auch leichtflüchtige Duftstoffe geeignet.

Verkapselungen sind dann sinnvoll, wenn Wirkstoffe geschützt und länger haltbar gemacht werden sollen, wenn sie gut in die Haut penetrieren, gleichmäßig verteilt und kontrolliert freigesetzt werden sollen.

Das Ziel einer Mikroverkapselung kann daher unterschiedlichen Zwecken dienen, wie dem des gesteuerten Freisetzungsverhaltens eines Wirkstoffs (controlled release), der Umhüllung flüssiger Substanzen, einer Maskierung oder Schutz des Kernmaterials, der Reduzierung der Flüchtigkeit sowie der Verbesserung der Verträglichkeit mit anderen Stoffen z. B. für die Compoundierung.

Unter dem Begriff "Mikrokapseln" werden erfindungsgemäß Partikel und Aggregate verstanden, die einen inneren Raum oder Kern enthalten, der mit einem festen, gelierten, flüssigen oder gasförmigen Medium gefüllt ist und von einer kontinuierlichen Hülle aus filmbildenden Polymeren umschlossen (verkapselt) sind. Diese Teilchen besitzen vorzugsweise kleine Abmessungen.

Zusätzlich können die mikroskopisch kleinen Kapseln ein oder mehr Kerne im kontinuierlichen Verkapselungsmaterial, bestehend aus einer oder mehreren Lagen, verteilt enthalten.

Bevorzugt sind einkernige Mikrokapseln mit einer kontinuierlichen Hülle.

Die Herstellung von Mikrokapseln wurde in der Literatur des Standes der Technik ausführlich beschrieben und ist mittels bekannten reaktiven und nichtreaktiven Verfahren zugänglich, wie Lösungsmittelverdampfung, Fällungsverfahren, Koazervation, Grenzflächenpolykondensation etc.

Die Lösungsmittelverdampfung wird zur Herstellung von Reservoir- und Matrixsystemen genutzt, dazu gehören u.a. Sprühtrocknung und Trommel-Coating.

Beim Fällungsverfahren wird das polymere Wandmaterial in einem mit Wasser mischbaren Lösungsmittel gelöst und der zu verkapselnde Wirkstoff darin dispergiert. Die Dispersion wird dann unter intensiver Durchmischung in die kontinuierliche wässrige Phase eingebracht.

Unter Koazervation versteht man die Separation einer kolloidalen Dispersion (flüssig/flüssig oder fest/flüssig) in einer Phase mit hohem Gehalt an flüssigem dispergierten Material (Koazervat) und eine Phase mit niedrigem Gehalt hervorgerufen durch äußere Einflüsse.

Bei der Technik der Grenzflächenpolykondensation wird im Gegensatz zu den anderen verwendeten Mikroverkapselungsverfahren wie Lösungsmittelverdampfung oder Koazervation, die sich bereits fertiger Polymere als Coatingmaterialien bedienen, die Schale erst im Verlauf des Verkapselungsprozesses aus den entsprechenden Monomeren gebildet.

Verkapselungsmaterialien sind üblicherweise natürliche, halbsynthetische oder synthetische anorganische und insbesondere organische Materialien.

Natürliche organische Materialien sind beispielsweise Gummiarabicum, Agar Agar, Agarose, Maltodextrine, Alginsäure bzw. ihre Salze, z. B. Natrium- oder Calciumalginat, Liposomen, Fette und Fettsäuren, Cetylalkohol, Collagen, Chitosan, Lecithine, Gelatine, Albumin, Schellack, Polysaccaride, wie Stärke oder Dextran, Cyclodextrine, Sucrose und Wachse.

Halbsynthetische Verkapselungsmaterialien sind unter anderem chemisch modifizierte Cellulosen, insbesondere Celluloseester und -ether, z. B. Celluloseacetat, Ethylcellulose, Hydroxypropylcellulose, Hydroxypropylmethylcellulose und Carboxymethylcellulose, sowie Stärkederivate, insbesondere Stärkeether und -ester.

Synthetische Verkapselungsmaterialien sind beispielsweise Polymere wie Aminoharze, Polyacrylate, Polyamide, Polyvinylalkohol oder Polyvinylpyrrolidon, Organopolysiloxane.

Die Modifizierung betrifft z. B. den Vernetzungsgrad der Polymere, die im wesentlichen die Durchlässigkeit der Hülle bestimmt, aber auch die chemische Zusammensetzung des Polymers, die für die Verträglichkeit zwischen Verkapselungs- und Kernmaterial verantwortlich ist.

Die Mikrokapseln können in Abhängigkeit vom Herstellungsverfahren hinsichtlich Form und Größe in weitem Rahmen variieren, sie sind jedoch bevorzugt näherungsweise knollen- oder kugelförmig und haben je nach den in ihrem Inneren enthaltenen Substanzen einen Durchmesser im Nanometerbereich (visuell nicht erkennbar, "unsichtbar") bis hin zum Millimeterbereich.

Unsichtbare Mikrokapseln haben vorzugsweise einen Durchmesser im Bereich von 20 bis 500 nm, vorzugsweise 50 bis 200 nm.

Die sichtbaren Kapseln sind größer als 500 Mikrometer im Durchmesser und aufgrund verkapselter Pigmente farbig. Man findet sie in Duschgelen, Haarpflegeprodukten und Zahncremes.

Die erfindungsgemäß verwendeten Mikrokapseln liegen bevorzugt im Bereich von 1 bis 1.000 µm insbesondere von 10 bis 200 µm. Einige der Verfahren zur Herstellung von Mikrokapseln zeichnen sich dadurch aus, dass drastische Herstellungsbedingungen mit Reaktionstemperaturen oberhalb von 100 °C benötigt werden. Derartige Verfahren sind zur Verkapselung kosmetischer Wirkstoffe nicht geeignet, denn oftmals wird unter solchen Bedingungen der zu verkapselnde Wirkstoff größtenteils bzw. in ungünstigen Fällen sogar vollständig zersetzt.

Die Freisetzung der Substanzen aus den Mikrokapseln erfolgt üblicherweise während der Anwendung der sie enthaltenden Zubereitungen durch Zerstörung der Hülle infolge mechanischer, thermischer, chemischer oder enzymatischer Einwirkung. Auch diese Öffnungsvarianten sind mitunter nicht ohne Auswirkung auf die wertvolle biologische Aktivität der verkapselten Inhaltsstoffe.

In kosmetischen Formulierungen für die Behandlung auch der normalen Haut, insbesondere aber empfindlicher, irritierter und ganz besonders in der Babypflege ist es aus naheliegenden Gründen jedoch oftmals problematisch oder unmöglich derartige mikroverkapselte Wirkstoffe anzuwenden.

Besonders wichtig für Anwendungen in kosmetischen Formulierungen ist der Grad der Penetration der mikroverkapselten Wirkstoffe in die Haut - verbunden mit einer Depotwirkung in der Hornschicht bzw. der Epidermis. Eine tiefe Penetration (transdermale Permeation) ist dabei eher pharmazeutischen Anwendungen vorbehalten.

Bei der Hautpflege muß weiterhin darauf geachtet werden, den Säureschutzmantel der Haut nicht durch ungeeignete Zusatzmittel zu schädigen sondern ihn zu erhalten und zu unterstützen, d.h. die "natürlichen" Umgebungsbedingungen weitgehend zu erhalten.

Die Oberfläche der Haut ist mit einem dünnen Film aus Hautfett, Schweiß und Aminosäuren überzogen. Die Bedeutung dieser sauren Eigenschaft der Hautoberfläche drückt sich im sogenannten "Säureschutzmantel" aus. Mit dem Begriff "Säureschutzmantel" ist gemeint, dass der Schutzfilm aus Fett und Wasser selbst wirkt wie eine ganz schwache Säure ("pH-Wert").

Neuere Forschungsergebnisse belegen, dass der saure pH-Wert der Hornschicht für die Bildung und Strukturierung der epidermalen Lipide und somit der Permeabilitätsbarriere eine essentielle Rolle spielt. Diese Untersuchungen zeigen, dass ein saures Milieu wichtig ist zur:
- Aktivierung der Enzyme für die Synthese wichtiger epidermaler Lipide,
- Ausbildung der Doppellipidmembran,
- Normalisierung der Hornschichtbarriere nach mechanischer oder chemischer Schädigung.

Durch nähere Betrachtung der Bestandteile des Hydrolipidfilms wird deutlich, warum dieser Schutzfilm 1928 erstmalig von Schade und Marchionini als Säureschutzmantel bezeichnet wurde:
- Schweiß enthält Milchsäure und verschiedene Aminosäuren,
- Talg enthält freie Fettsäuren,
- Aus dem Verhornungsprozess stammen Aminosäuren und Pyrrolidoncarbonsäure.

Die oberste Hautschicht ist aus übereinander geschichteten Zellen aufgebaut, die locker vergleichbar wie Dachziegel übereinander liegen. Das Material für diese Schicht ist eigentlich Hautabfall aus toten und platten Hornzellen. Diese sind durch Hautfette oder Lipide und Feuchtigkeit verklebt. Weil Fett Wasser abweist, wirkt diese Fett-Feuchtigkeits-Mischung in der Oberhaut nach außen wie ein Regenmantel. Gleichzeitig verhindert sie, dass unsere Haut zu viel Feuchtigkeit von innen durch Körperwärme verdunstet. Wasserlösliche Schadstoffe haben kaum eine Chance, diese Barriere zu durchdringen. Das Gleiche gilt aber auch für wasserlösliche Pflegestoffe. Fettlöslichen Wirkstoffen gelingt es leichter, in die Haut einzudringen.

Anforderungen die idealer Weise an ein Verkapselungssystem für kosmetische Wirkstoffe gestellt werden, sind daher vielfältig. Neben einem schonenden und schnellen Einschlussverfahren, welches einfach durchführbar sein soll und sich zur Herstellung von Mikrokapseln mit konstanter Qualität eignet, soll der zu verkapselnde Wirkstoff möglichst vollständig umhüllt sein, weil nur dann ein hinlänglicher Schutz gegeben ist. Vorzugsweise geschieht die Herstellung der Mikrokapseln in einem einfachen Einschrittverfahren und benutzt als Wandmaterial kommerziell erhältliche Polymere, die sich durch eine definierte chemische Zusammensetzung auszeichnen. Bei der Wahl des Polymermaterials ist weiterhin zu berücksichtigen, dass keine unerwünschten Hautreaktionen hervorgerufen werden und dass die Art des Freisetzungsmechanismus so eingestellt werden kann, dass eine Beeinträchtigung des Hautsäureschutzmantels nicht erfolgt.

Eine Aufgabe der vorliegenden Erfindung hat darin bestanden, kosmetische Zubereitungen für die Behandlung der Haut welche die Wirkstoffe in einer Mikroverkapselung enthalten bereit zu stellen welche eine breite Vielfalt der bereits erwähnten Anforderungskriterien erfüllen und den Wirkstoff nach Aufbringen auf die Haut kontinuierlich freisetzen ohne den Säureschutzmantel der Haut zu beeinträchtigen.

Besondere Vorteile bietet dabei ein Polymersystem, in welchem kosmetische Wirkstoffe nur geringe Löslichkeit aufweisen, da in einem solchen Polymergemisch der Wirkstoff ein hohes Bestreben hat, das Polymer zu verlassen. Die geringe Dichte des Systems sorgt zusätzlich für kurze Diffusionswege.

Aus der japanischen Patentanmeldung JP-A-06-105069 ist ein Verfahren zur Herstellung von pH-sensitiven Mikrokapseln bekannt. In diesem Zusammenhang ist angegeben, dass das pH-empfindliche Polymer zunächst gelöst wird und dann mit dem zu verkapselnden Material und einem porösen Trägermaterial, wie z.B. Silica, dispergiert wird. Die so erhaltene Suspension wird in ein Dispersionsmedium gegeben und unter Abdampfen des Lösungsmittels oder durch Phasentrennung im Dispersionsmedium entstehen Mikrokapseln mit Durchmessern zwischen 0,001 bis 1.000 Mikrometern. Es wird darauf hingewiesen, dass die unzureichende Stabilität der Mikrokapsel durch den Zusatz eines porösen Trägermaterials verbessert wird. Da der aktive Wirkstoff in die Poren des Trägermaterials aufgenommen wird, kann keine innere Zersetzung der Mikrokapsel bzw. keine Beeinträchtigung der kosmetischen Formulierung durch den Wirkstoff zustande kommen. Als einsetzbare Polymertypen werden im Rahmen einer allgemeinen Aufzählung sowohl alkali- als auch säure-labile Polymere erwähnt.

Aus der japanischen Patentanmeldung JP-A-07-096166 ist ein Verfahren zur Herstellung von pH-sensitiven Mikrokapseln bekannt, deren Wandmaterialien aus Polymethylenchlorid bestehen sollen.

In der US-A-5 364 634 wird eine Zusammensetzung aus pHempfindlichen Mikrokapseln und einem adhäsiven Träger zur oralen Verwendung beschrieben.

Die FR-A-2 753 639 beschreibt ein Verfahren zur Herstellung von Mikrokapseln unter Verwendung von in superkritischem CO₂ unlöslichen Polymeren.

Rowe R.C. et al. "Handbook of Pharmaceutical exipients", 4. Auflage, London, Pharmaceutical Press, 2003, Seite 462-468 beschreibt die chemische Zusammensetzung einiger Polymethacrylate sowie ihre Löslichkeiten in den verschidenen pH-Bereichen und die Verwendung dieser Verbindungen als filmbildende Materialien für Kapseln und Tabletten.

Überraschenderweise wurde gefunden, dass sich durch die Verwendung von Eudragit® E100, einem Copolymer auf Basis von 2-Dimethylaminoethylmethacrylat, Methylmethacrylat und n-Butylmethacrylat, als Verkapselungsmaterial für die Wirkstoffe, Mikrokapseln für die Einarbeitung in kosmetischen Formulierungen herstellen lassen, die ohne die Verwendung zusätzlicher Agenzien und Trägermaterialien und ohne die Anwendung mechanischer Energie zum Durchlässigmachen des Kapselwandmaterials herstellbar sind.

Es wurde ferner festgestellt, dass auch durch Abmischungen dieses Basispolymers mit beliebigen anderen Polymeren das pH-gesteuerte Freisetzungsverhalten erhalten bleibt, insofern der Anteil an Basispolymer über 20 Gew.-% ausmacht. Durch das Abmischen mit anderen, vorzugsweise mit ionisierbaren Gruppen funktionalisierten Polymeren können Eigenschaften wie Bioabbaubarkeit, das Freisetzungsverhalten der aktiven Wirkstoffe und auch die Herstellkosten günstig beeinflusst werden.

Ein Gegenstand der Erfindung sind somit Kosmetische Zubereitungen für die Behandlung der Haut, enthaltend einen oder mehrere Wirkstoffe in einer Mikroverkapselung deren Verkapselungsmaterial aus Copolymeren auf Basis von 2-Dimethylaminoethylmethacrylat, Methylmethacrylat und n-Butylmethacrylat besteht und im pH-Bereich der Haut durchlässig ist und/oder abgebaut wird, dadurch gekennzeichnet, dass das Kernmaterial frei von porösen Materialien ist und deren Verkapselungsmaterial sich bei pH-Werten von zwischen ca. 4,6 bis ca. 6,0 zersetzt.

Weitere Gegenstände der Erfindung sind durch die Ansprüche gekennzeichnet.

In einer bevorzugten Ausführungsform der Erfindung enthalten die Mittel Mikrokapseln in Mengen von 0,1 bis 10 Gew.-%, insbesondere 0,2 bis 8 Gew.-%, besonders bevorzugt 0,5 bis 5 Gew.-%.

Die erfindungsgemäß eingesetzten Verkapselungsmaterialien sind Copolymere auf Basis von 60 bis 40 Gew.-% 2-Dimethylaminoethylmethacrylat, 20 bis 30 Gew.-% Methylmethacrylat und 20 bis 30 Gew.-% n-Butylmethacrylat und Copolymere auf der Basis von jeweils 50 Gew.-% Methylmethacrylat und Ethylacrylat.

Diese Verbindungen und ihre Herstellung sind beschrieben in der DE-B-1 617 751 sowie der EP-A-0 181 515.

Die entsprechenden Handelsprodukte sind unter dem Markennamen EUDRAGIT® der Firma Röhm GmbH, Darmstadt erhältlich.

Durch Variation des Copolymerisationsgrads kann die Zusammensetzung des Polymers so eingestellt werden, dass das resultierende Verkapselungsmaterial oberhalb von pH 5 löslich, quellbar und durchlässig wird.

Die erfindungsgemäß bevorzugt verwendeten Copolymere auf Basis von 50 Gew.-% 2-Dimethylaminoethylmethacrylat, 25 Gew.-% Methylmethacrylat und 25 Gew.-% n-Butylmethacrylat sind dadurch gekennzeichnet, dass sie mittlere Molmassen von 50.000 bis 250.000 g/mol aufweisen, wobei die vorzugsweise verwendeten Materialien solche im Bereich von 100.000 bis 200.000 g/mol, insbesondere 130.000 bis 170.000 g/mol aufweisen sollen.

Es ist zudem möglich, dieses Basis-Copolymer in Abmischung mit anderen natürlichen oder synthetischen Polymeren zu verwenden, solange sichergestellt ist, dass die pH-gesteuerte Öffnung der resultierenden Gemische erhalten bleibt.

Typische Beispiele für Wirkstoffe, wie sie im Bereich der kosmetischen Zubereitungen eingesetzt werden sind Tenside, kosmetische Öle, Perlglanzwachse, Stabilisatoren, antimikrobielle Wirkstoffe, entzündungshemmende Wirkstoffe, Pflanzen-, Hefe- und Algenextrakte, Vitamine, Vitaminderivate und -komplexe, Aminosäuren und Aminosäurederivate, bioaktive Lipide wie Cholesterol, Ceramide und Pseudoceramide, Deodorantien, Antitranspirantien, Antischuppenmittel, UV-Lichtschutzfaktoren, Antioxidantien, Konservierungsmittel, Insektenrepellentien, Selbstbräuner, Tyrosinase-Inhibitoren (Depigmentierungsmittel), Parfümöle und Farbstoffe. Bevorzugt eingesetzte Wirkstoffe sind diejenigen, welche in nicht verkapselter Form entweder in Formulierungen nicht stabil einarbeitbar sind oder zumindest nicht über längere Lagerzeiträume stabil bleiben.

Die kosmetischen Zubereitungen für die Behandlung der Haut sind praxisübliche Formulierungen, welche die für die jeweiligen Anwendungszwecke typischen Bestandteile in den üblichen Mengen enthalten. Diese Formulierungen sind dem Fachmann bekannt und können so verwendet werden, vorausgesetzt der pH-Wert liegt außerhalb des Bereichs in dem die Zerstörung des verwendeten Verkapselungsmaterials auftritt.

Die folgenden Beispiele sollen den Gegenstand der Erfindung näher erläutern:

### Polymer:

Copolymer auf Basis von 50 Gew.-% 2-Dimethylaminoethylmethacrylat, 25 Gew.-% Methylmethacrylat und 25 Gew.-% n-Butylmethacrylat und einem mittleren Molgewicht von ca. 150.000 g/mol (EUDRAGIT® E 100, Röhm GmbH):

### Beispiel 1:

Es werden 5 g Polymer in 30 ml Aceton gelöst. Anschließend werden 0,1 g Aluminiumtristearat als Emulgator und 0,5 g Tocopherol als Wirkstoff hinzugegeben. Diese Lösung wird für 20 Minuten bei 10 °C und 250 U/min gerührt und anschließend zu 200 ml 10 °C kaltem Paraffinöl gegeben. Die entstandene Reaktionslösung wird für weitere 4 Stunden bei 190 U/min bzw. 500 U/min gerührt anschließend abfiltriert und mit 50 ml n-Hexan gewaschen. Die entstandenen Kugeln werden bei Raumtemperatur getrocknet.

### Ergebnis:

Es entstehen gleichmäßig geformte Kugeln die einen mittleren Durchmesser von 600 µm aufweisen. Die Kugeln kleben nicht zusammen und liegen somit einzeln vor. Ein pH-gesteuertes Öffnen der Kapseln ist mit Salzsäure (pH-Wert 5,5), sowie in einer Pufferlösung welche auf einen pH-Wert von 5,0 eingestellt ist, möglich. Durch Zugabe des Puffers zu den Kugeln ist nach etwa 15 Minuten unter dem Mikroskop ein Austreten des Wirkstoffs zu erkennen. Nach weiteren 45 Minuten löst sich die Kugel langsam auf und der Wirkstoff wird deutlich sichtbar.

### Beispiel 2:

Es werden 1 g Polymer in 30 ml Aceton gelöst. Anschließend werden 0,1 g Aluminiumtristearat als Emulgator und 0,5 g Liponsäure als Wirkstoff hinzugegeben. Diese Lösung wird für 20 Minuten bei 10 °C und 250 U/min gerührt und anschließend zu 200 ml 10 °C kaltem Paraffinöl gegeben. Die entstandene Reaktionslösung wird für weitere 4 Stunden bei 200 U/min gerührt anschließend abfiltriert und mit 50 ml n-Hexan gewaschen. Die entstandenen Kugeln werden bei Raumtemperatur getrocknet.

### Ergebnis:

Es entstehen gleichmäßig geformte Kugeln die einen mittleren Durchmesser von 200 µm aufweisen. Die Kugeln kleben nicht zusammen und liegen somit einzeln vor. Ein pH-gesteuertes Öffnen der Kapseln ist mit Salzsäure (pH-Wert 5,5), sowie mit Puffer (pH-Wert 5,0) möglich. Durch Zugabe des Puffers zu den Kugeln ist nach etwa 10 Minuten unter dem Mikroskop ein Austreten des Wirkstoffs zu erkennen. Nach weiteren 30 Minuten löst sich die Kugel langsam auf und der Wirkstoff wird deutlich sichtbar.

### Beispiel 3:

Es werden 5 g Polymer in 30 ml Aceton gelöst. Anschließend werden 0,5 g Emulgator (z.B. Aluminiumtristearat) und 0,5 g Menthol hinzugegeben. Diese Lösung wird für 20 Minuten bei 10 °C und 250 U/min gerührt und anschließend zu 200 ml 10 °C kaltem Paraffinöl gegeben. Die entstandene Reaktionslösung wird für weitere 4 Stunden bei 250 U/min gerührt anschließend abfiltriert und mit 50 ml n-Hexan gewaschen. Die entstandenen Kugeln werden bei Raumtemperatur getrocknet.

### Ergebnis:

Es entstehen gleichmäßig geformte Kugeln die einen mittleren Durchmesser von 150 µm aufweisen. Die Kugeln kleben nicht zusammen und liegen somit einzeln vor. Ein pH-gesteuertes Öffnen der Kapseln ist mit Salzsäure (pH-Wert 5,5), sowie mit Puffer (pH-Wert 5,0) möglich. Durch Zugabe des Puffers zu den Kugeln ist nach etwa 10 Minuten unter dem Mikroskop ein Austreten des Wirkstoffs zu erkennen. Nach weiteren 30 Minuten löst sich die Kugel langsam auf und der Wirkstoff wird deutlich sichtbar.

### Beispiel 4:

Es werden 2,5 g Polymer mit 2,5 g Poly(dl-lactid-co-gly-colid) in 30 ml Aceton gelöst. Anschließend werden 0,1 g Emulgator (z.B. Aluminiumtristearat) und 0,5 g Vitamin E hinzugegeben. Diese Lösung wird für 20 Minuten bei 10 °C und 250 U/min gerührt und anschließend zu 200 ml 10 °C kaltem Paraffinöl gegeben. Die entstandene Reaktionslösung wird für weitere 4 Stunden bei 250 U/min gerührt anschließend abfiltriert und mit 50 ml n-Hexan gewaschen. Die entstandenen Kugeln werden bei Raumtemperatur getrocknet.

### Ergebnis:

Es entstehen gleichmäßig geformte Kugeln die einen mittleren Durchmesser von 300 µm aufweisen. Die Kugeln kleben nicht zusammen und liegen somit einzeln vor. Ein pH-gesteuertes Öffnen der Kapseln ist mit Salzsäure (pH-Wert 5,5), sowie mit Puffer (pH-Wert 5,0) möglich. Durch Zugabe des Puffers zu den Kugeln ist nach etwa 12 Minuten unter dem Mikroskop ein Austreten des Wirkstoffs zu erkennen. Nach weiteren 40 Minuten löst sich die Kugel langsam auf und der Wirkstoff wird deutlich sichtbar.

pH-gesteuertes Freisetzten des Wirkstoffs mit Pufferlösung (in vitro):

Die entstandenen Kugeln aus Versuch 1 werden in Pufferlösung pH 5,0 (Merck) gegeben und nach unterschiedlichen Zeiten photometrisch gemessen (Wellenlänge 332 nm). Die Extinktion ist direkt proportional dem freigesetzten prozentualen Anteil des Wirkstoffs.

### Einwaage:

0,5 g Kugeln/50 ml Pufferlösung

| Zeit | Freisetzung % |
|---|---|
| 20 sec | 53,5 |
| 1 min | 69,0 |
| 2 min | 85,9 |
| 2,30 min | 89,4 |
| 3 min | 91,7 |
| 3,30 min | 96,4 |
| 4 min | 100 |

pH-gesteuertes Freisetzten des Wirkstoffs auf der Haut (in vivo) :

Es wurde eine klassische Hautpflege-Creme auf Basis einer W/O-Emulsion hergestellt. Hierzu wurde eine Ölphase aus 45,6 g Paraffinöl und 2,4 g ABIL EM 90 (Goldschmidt) vorgelegt und mit einem MIG-Rührer bei 450 U/min gerührt. In diese wurde eine Wasserphase aus 147,2 g Wasser, 4,0 g Glycerin und 0,8 g NaCl innerhalb von 3 min hinzugegeben und danach 3 min bei 1.300 U/min homogenisiert. Abschliessend wurde mit Zitronensäure auf einen pH-Wert von 6,5 eingestellt und in die fertige Creme 1 Gew.-% der erfindungsgemäßen Mikrokapseln mit Liponsäure als Wirkstoff (hergestellt wie in Beispiel 2 beschrieben) hineingerührt. Diese Formulierung wurde bei Raumtemperatur und in der Wärme bei 40 °C über einen Zeitraum von insgesamt 2 Monaten gelagert. Während dieser Zeit wurden im Wochenabstand Proben entnommen, die Form der Kapseln mikroskopisch untersucht und nach Filtration der Kapseln die Creme-Formulierung mittels HPLC-Analytik auf ihren Gehalt an freigesetzter Liponsäure kontrolliert. Als Ergebnis zeigte sich, dass unter den beschriebenen Bedingungen die Mikrokapseln über die gesamte Lagerzeit stabil blieben und ebenso kein Austritt von Wirkstoff in die Creme zu beobachten war. Die pH-induzierte Öffnung der Kugeln wurde überprüft, indem die Creme auf die Haut von insgesamt 6 Testpersonen aufgetragen wurde und die Stellen anschließend mit einem Klebeband überdeckt wurden.

Nach einer Einwirkzeit von einer Stunde wurde das Klebeband mit den daran anhaftenden Kapseln bzw. Kapselrückständen von der Haut entfernt und unter dem Mikroskop betrachtet. Unter dem Mikroskop war deutlich zu erkennen, dass eine Öffnung der Polymerkapseln unter den sauren pH-Bedingungen der Haut stattgefunden hat.

Ein identisches Ergebnis konnte auch dadurch erreicht werden, dass größere Kapseln direkt der Creme entnommen wurden und ebenfalls nach Klebeband-Fixierung auf der Haut nach einer Stunde von der Haut entfernt wurden und danach mikroskopisch untersucht wurden. Auch hier zeigte sich der erhoffte Effekt, da kein intaktes Kapselmaterial mehr zu finden war.

## Patentansprüche

1. Kosmetische Zubereitungen für die Behandlung der Haut, enthaltend einen oder mehrere Wirkstoffe in einer Mikroverkapselung deren Verkapselungsmaterial aus Copolymeren auf Basis von 2-Dimethylaminoethylmethacrylat, Methylmethacrylat und n-Butylmethacrylat besteht und im pH-Bereich der Haut durchlässig ist und/oder abgebaut wird, **dadurch gekennzeichnet, dass** das Kernmaterial frei von porösen Materialien ist und deren Verkapselungsmaterial sich bei pH-Werten von zwischen ca. 4,6 bis ca. 6,0 zersetzt.

2. Kosmetische Zubereitungen gemäß Anspruch 1, **dadurch gekennzeichnet, dass** die Zubereitungen zusätzlich nicht-mikroverkapselte Wirkstoffe enthalten.

3. Kosmetische Zubereitungen gemäß Anspruch 1 und/oder 2, **dadurch gekennzeichnet, dass** sie 0,1 bis 10 Gew.-%, bezogen auf die Gesamtformulierung, an Mikrokapseln enthalten.

4. Kosmetische Zubereitungen nach mindestens einem der Ansprüche 1 bis 3, **dadurch gekennzeichnet, dass** sie Mikrokapseln enthalten, deren Verkapselungsmaterial aus Copolymeren auf Basis von 60 bis 40 Gew.-% 2-Dimethylaminoethylmethacrylat, 20 bis 30 Gew.-% Methylmethacrylat und 20 bis 30 Gew.-% n-Butylmethacrylat besteht.

5. Kosmetische Zubereitungen nach mindestens einem der Ansprüche 1 bis 4, **dadurch gekennzeichnet, dass** sie Mikrokapseln enthalten, deren Verkapselungsmaterial aus Copolymeren auf Basis von 2-Dimethylaminoethylmethacrylat, Methylmethacrylat und n-Butylmethacrylat mit einer mittleren Molmasse von 50.000 bis 250.000 g/mol besteht.

6. Kosmetische Zubereitungen nach mindestens einem der Ansprüche 1 bis 5, **dadurch gekennzeichnet, dass** zusätzliche Anteile an Verkapselungsmaterialien ausgewählt aus der Gruppe, die gebildet wird von Gummiarabicum, Agar Agar, Agarose, Maltodextrine, Alginsäure, Alginaten, Fetten, Fettsäuren, Cetylalkohol, Collagen, Chitosan, Lecithin, Gelatine, Albumin, Schellack, Polysaccariden, Cellulosen, Celluloseestern, Celluloseethern, Stärkeethern, Stärkeestern, Polyacrylaten, Polyamiden, Polyvinylalkoholen und Polyvinylpyrrolidon mitverwendet werden.

7. Kosmetische Zubereitungen nach mindestens einem der Ansprüche 1 bis 6, **dadurch gekennzeichnet, dass** sie näherungsweise sphärische wie knollen- oder kugelförmige Mikrokapseln enthalten, deren Durchmesser von 1 bis 1.000 µm beträgt.

## Claims

1. Cosmetic preparations for the treatment of skin, comprising one or more active ingredients in a microencapsulation whose encapsulation material consists of copolymers based on 2-dimethylaminoethyl methacrylate, methyl methacrylate and n-butyl methacrylate and is permeable and/or is degraded in the pH range of the skin, **characterized in that** the core material is free from porous materials and whose encapsulation material decomposes at pH values of between about 4.6 and about 6.0.

2. Cosmetic preparations according to Claim 1, **characterized in that** the preparations additionally comprise non-microencapsulated active ingredients.

3. Cosmetic preparations according to Claim 1 and/or 2, **characterized in that** they comprise 0.1 to 10% by weight, based on the total formulation, of microcapsules.

4. Cosmetic preparations according to at least one of Claims 1 to 3, **characterized in that** they comprise microcapsules whose encapsulation material consists of copolymers based on 60 to 40% by weight of 2-dimethylaminoethyl methacrylate, 20 to 30% by weight of methyl methacrylate and 20 to 30% by weight of n-butyl methacrylate.

5. Cosmetic preparations according to at least one of Claims 1 to 4, **characterized in that** they comprise microcapsules whose encapsulation material consists of copolymers based on 2-dimethylaminoethyl methacrylate, methyl methacrylate and n-butyl methacrylate with an average molar mass of from 50 000 to 250 000 g/mol.

6. Cosmetic preparations according to at least one of Claims 1 to 5, **characterized in that** additional fractions of encapsulation materials chosen from the group formed by gum arabic, agar agar, agarose, maltodextrins, alginic acid, alginates, fats, fatty acids, cetyl alcohol, collagen, chitosan, lecithin, gelatin, albumin, shellac, polysaccarides, celluloses, cellulose esters, cellulose ethers, starch ethers, starch esters, polyacrylates, polyamides, polyvinyl alcohols and polyvinylpyrrolidone are co-used.

7. Cosmetic preparations according to at least one of Claims 1 to 6, **characterized in that** they comprise approximately spherical, such as bulb-shaped or ball-shaped, microcapsules whose diameter is from 1 to 1 000 µm.

## Revendications

1. Préparations cosmétiques pour le traitement de la peau, contenant une ou plusieurs substances actives dans une microencapsulation dont le matériau d'encapsulation est constitué de copolymères à base de méthacrylate de 2-diméthylaminoéthyle, méthacrylate de méthyle et méthacrylate de n-butyle et est dégradé et/ou perméable dans l'intervalle de pH de la peau, **caractérisées en ce que** le matériau du noyau est exempt de matières poreuses et dont le matériau d'encapsulation se décompose à des pH compris entre environ 4,6 et environ 6,0.

2. Préparations cosmétiques selon la revendication 1, **caractérisées en ce que** les préparations contiennent en outre des substances actives non microencapsulées.

3. Préparations cosmétiques selon la revendication 1 et/ou la revendication 2, **caractérisées en ce qu'**elles contiennent de 0,1 à 10 % en poids, par rapport à la composition totale, de microcapsules.

4. Préparations cosmétiques selon au moins l'une des revendications 1 à 3, **caractérisées en ce qu'**elles contiennent des microcapsules dont le matériau d'encapsulation est constitué de copolymères à base de 60 à 40 % en poids de méthacrylate de 2-diméthylaminoéthyle, 20 à 30 % en poids de méthacrylate de méthyle et 20 à 30 % en poids de méthacrylate de n-butyle.

5. Préparations cosmétiques selon au moins l'une des revendications 1 à 4, **caractérisées en ce qu'**elles contiennent des microcapsules dont le matériau d'encapsulation est constitué de copolymères à base de méthacrylate de 2-diméthylaminoéthyle, méthacrylate de méthyle et méthacrylate de n-butyle ayant une masse moléculaire moyenne de 50 000 à 250 000 g/mole.

6. Préparations cosmétiques selon au moins l'une des revendications 1 à 5, **caractérisées en ce qu'**on utilise en même temps des quantités supplémentaires de matériaux d'encapsulation choisis dans le groupe constitué par la gomme arabique, l'agar-agar, l'agarose, les maltodextrines, l'acide alginique, des alginates, des graisses, des acides gras, l'alcool cétylique, le collagène, le chitosane, la lécithine, la gélatine, l'albumine, la gomme-laque, des polysaccharides, les celluloses, les esters de cellulose, les éthers de cellulose, les éthers d'amidon, les esters d'amidon, les polyacrylates, les polyamides, les poly(alcool vinylique)s et la polyvinylpyrrolidone.

7. Préparations cosmétiques selon au moins l'une des revendications 1 à 6, **caractérisées en ce qu'**elles contiennent des microcapsules approximativement sphériques, telles que des microcapsules globulaires ou en forme de billes, dont le diamètre va de 1 à 1 000 µm.
